(19) Europäisches Patentamt
European Patent Office
Office européen des brevets

(11) **EP 4 647 067 A1**

(12) **EUROPEAN PATENT APPLICATION**

(43) Date of publication:
**12.11.2025** Bulletin 2025/46

(21) Application number: **24382505.6**

(22) Date of filing: **08.05.2024**

(51) International Patent Classification (IPC):
***A61K 9/16*** *(2006.01)* ***A61K 9/20*** *(2006.01)*
***A61K 36/889*** *(2006.01)*

(52) Cooperative Patent Classification (CPC):
**A61K 9/1652; A61K 9/1694; A61K 9/2059;**
**A61K 36/889;** A61K 2236/33; A61K 2236/35;
A61K 2236/37

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Tradichem Industrial Services, S.L.**
**28108 Alcobendas (ES)**

(72) Inventors:
- **MARAÑÓN MAROTO, José Ángel**
  **28240 HOYO DE MANZANARES (ES)**
- **MORENO PUENTE, Patricia**
  **28052 MADRID (ES)**
- **RAMÍREZ BORREGO, Carlos**
  **28052 MADRID (ES)**
- **LOZANO MARTÍN, Cristina**
  **28521 RIVAS VACIAMADRID (ES)**

(74) Representative: **Sugrañes, S.L.P.**
**Calle Provenza 304**
**08008 Barcelona (ES)**

(54) **GRANULAR SERENOA REPENS EXTRACT**

(57) The invention relates to a process for preparing *Serenoa repens* extract granules comprising roller compaction of a *Serenoa repens* solid extract. The invention also relates to the granular *Serenoa repens* obtainable with such process. The invention also relates to the use said granular product for the preparation of a pharmaceutical composition, to a pharmaceutical composition containing it and to a process for preparing *Serenoa repens* compositions using said granules. Additionally, the invention further relates to said *Serenoa repens* pharmaceutical compositions for use in therapy.

FIGURE 2

**Description**

TECHNICAL FIELD

The present invention relates to granular *Serenoa repens* extract of improved properties and to a process for preparing it.

BACKGROUND ART

*Serenoa repens* extract is an herbal medicinal substance, namely, it is a lipidosterolic extract of dried ripe fruit of *Serenoa repens* (W. Bartram) Small, also known as saw palmetto.

*Serenoa repens* extracts can be prepared using hexane or ethanol as solvents for extraction or by supercritical fluid extraction based on the use of carbon dioxide in supercritical phase. The main components of these lipidosterolic extracts are fatty acid esters and free fatty acids, sterols and fatty alcohols (Saw Palmetto, in: Herbal Medicines. Third edition. J Barnes, L.A. Anderson and J.D. Phillipson, editors; Pharmaceutical Press 2007: 521-529).

This herbal substance is an effective remedy for the treatment of benign prostatic hyperplasia (BPH) and the management of associated complications. Its action is comparable with that of the synthetic pharmacological molecules currently used for this pathology, such as finasteride or tamsulosin, but it has the advantage that is harmless and has reduced side effects (Annunziata et al., Saw Palmetto (Serenoa repens), Nonvitamin and Nonmineral Nutritional Supplements, 401-402, doi:10.1016/b978-0-12-812491-8.00053-9).

There is, therefore, a growing interest in the preparation of pharmaceutical compositions comprising *Serenoa repens* extract as active ingredient. However, said extracts are not generally suitable for preparing pharmaceutical compositions, particularly, for preparing solid pharmaceutical compositions and, more particularly, tablets by direct compression.

*Serenoa repens* extract is typically an oil, whose formulation options are, therefore, limited. For preparing solid oral pharmaceutical dosage forms, in particular, it can be formulated as soft gelatin capsules or, alternatively, needs to be previously adsorbed on some solid support, such as maltodextrin or alginate, among others, as disclosed, for example, in Lopedota et al., From oil to microparticulate by prilling technique: Production of polynucleate alginate beads loading Serenoa repens oil as intestinal delivery systems, Int. J. Pharm. 2021, 599: 120412. Another option disclosed in the art is mixing the *Serenoa repens* lipidosterolic extract with molten high molecular polyethylene glycol as carrier, filling hard gelatin capsules with said mixture and allowing it to cool and solidify, as disclosed, for example, in WO-A-2012/017022.

Solid extracts of *Serenoa repens* are commercially available, where the lipidosterolic oily extract, obtained through different extraction methods, is combined with a carrier, and said solid extracts are more convenient for preparing solid dosage forms than the oily extracts. Nevertheless, these *Serenoa repens* solid extract products are generally poorly flowable powders and, therefore, also have important drawbacks to be formulated into suitable solid pharmaceutical dosage forms.

Thus, for example, the article Tomar et al., Hicel™ silicified microcrystalline cellulose, versatile excipient for nutraceutical herbal tablet formulation, Int. J. Dev. Res., 2019, 9(6): 28009-104, discloses that the formulation of herbal extracts such as *Serenoa repens,* is challenging due to the high dose required, its bitter taste and its very poor flowability and compressibility. These inherent poor tableting properties hamper the manufacturing of tablets using direct compression method. In the formulation examples provided in this article, it is shown that 265 mg tablets of *Serenoa repens* prepared with conventional excipients (microcrystalline cellulose, colloidal silicon dioxide, croscarmellose sodium and purified talc, formulation F5) show important drawbacks derived from the poor flowability of the powder blend. Consequently, the tablets prepared therewith showed very poor weight uniformity, low hardness and high friability. The solution proposed in this article to provide direct compression *Serenoa repens* tablets of optimal characteristics is to use a special formulation excipient, namely, silicified microcrystalline cellulose.

In view of the poor tableting properties of *Serenoa repens,* there is the need of simple and efficient methods for preparing solid pharmaceutical dosage forms, particularly tablets, comprising *Serenoa repens* as active ingredient.

SUMMARY OF THE INVENTION

The object of the present invention is a process for the preparation of granular *Serenoa repens* extract.

Another aspect of the invention is the granular *Serenoa repens* extract obtainable with such process.

Another aspect of the invention is the use of said granular *Serenoa repens* extract for the preparation of pharmaceutical compositions.

Another aspect of the invention is a pharmaceutical composition comprising said granular *Serenoa repens* extract.

Another aspect of the invention is a process for the preparation of a pharmaceutical composition comprising the use of said granular *Serenoa repens* extract.

Another aspect of the invention is a pharmaceutical composition comprising said granular *Serenoa repens* extract for use in therapy.

## DESCRIPTION OF THE DRAWINGS

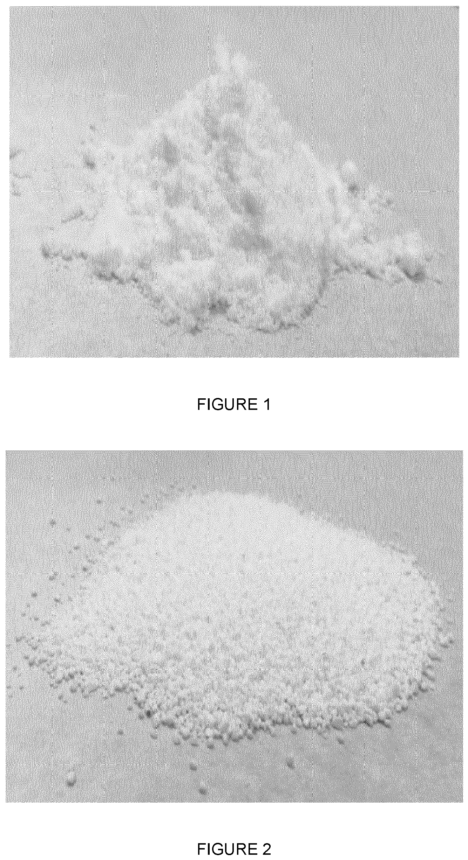

Figure 1 shows a picture of *Serenoa repens* solid extract starting material used in Example 1 and Figure 2 shows a picture of the granular *Serenoa repens* extract obtained in Example 1.

## DETAILED DESCRIPTION OF THE INVENTION

The object of the present invention is a process for the preparation of *Serenoa repens* extract granules comprising roller compaction of a *Serenoa repens* solid extract.

The authors of the present invention have found that by subjecting *Serenoa repens* powder extract to a process of roller compaction, granules of *Serenoa repens* can be obtained having improved flowability parameters, which allows for preparing tablets therewith by simple direct compression technique and using customary formulation excipients.

Along the present description, as well as in the claims, singular expressions, generally preceded by the articles "a", "an" or "the", are intended to include the plural forms as well, unless the context clearly indicates otherwise.

The terms "about" or "approximately" referred to amounts, as used herein, are meant to include the exact amount and also a certain deviation around the stated amount, namely of $\pm 5\%$.

Unless otherwise indicated, the stated percentages are always weight percentages.

The numerical ranges disclosed herein, expressed as "from ... to ..." or as "between ... and ...", are meant to include any number falling within the ranges and also the lower and upper limits.

### *Serenoa repens* solid extract

The terms *Serenoa repens* solid extract and *Serenoa repens* powder extract are used interchangeably herein and they mean a powdery product typically comprising the oily active substance, i.e., the lipidosterolic oily extract of *Serenoa repens*, combined with a suitable solid pharmaceutically acceptable carrier.

*Serenoa repens* extracts are obtained by extraction of dried ripe fruit of *Serenoa repens* (W. Bartram) Small. Synonyms of the plant include *Sabal serrulata* (Mich.) Nutall ex Schult and *Serenoa serrulata* Hook (Eur. Pharm 2012). It is also commonly known as saw palmetto. The *Serenoa repens* extract is obtained as an oil and is commonly referred to as lipidosterolic extract, due to its composition, rich in lipids and sterols.

Within the present description, the terms *Serenoa repens* extract, *Serenoa repens* fruit extract, *Serenoa serrulata* extract, *Serenoa serrulata* fruit extract, saw palmetto extract or saw palmetto fruit extract can be used interchangeably, and all designate the extract of dried ripe fruit of *Serenoa repens* (W. Bartram) Small.

The lipidosterolic oily *Serenoa repens* extract can be obtained through extraction of dried ripe fruit of *Serenoa repens* (W. Bartram) Small, according to any standard extraction process, typically, dried berries are ground into a powder and are extracted using n-hexane, ethanol or supercritical $CO_2$ as extraction solvent/fluid, as disclosed, for example, in Assessment report on *Serenoa repens* (W. Bartram) Small, fructus. Final. European Medicines Agency, 24.11.2015; or in Hänsel et al., Hagers Handbuch der Pharmazeutischen Praxis: Drogen P-Z, Springer-Verlag, Berlin 1994.

In an embodiment, the *Serenoa repens* extract is obtained with an extraction fluid selected from *n*-hexane and supercritical $CO_2$. In a more particular embodiment, *Serenoa repens* is obtained by supercritical $CO_2$ extraction. Supercritical $CO_2$ extraction can be performed, for example, as disclosed in EP-A-250953, or in GB-A-2343452, or in WO-A-2023/023459. Extraction with n-hexane is disclosed, for example, in FR-A-2480754.

The main components of *Serenoa repens* fruit are carbohydrates, sterols, flavonoids, triglycerides and fatty acids, and other minor components, such as volatile oils, anthranilic acid, carotene, resin, and tannin (Assessment report on *Serenoa repens* (W. Bartram) Small, fructus. Final. European Medicines Agency, 24.11.2015). On the other hand, the composition of the lipidosterolic oily extract obtained therefrom varies, depending on extraction conditions and *Serenoa repens* plant source used. It is a complex mixture comprising free and esterified fatty acids as major components, together with phytosterols and fatty alcohols (Ham et al., A study of the physical and chemical properties of saw palmetto berry extract, Science Direct Working Paper No S1574-0331 (04)70214-3).

Any lipidosterolic oily *Serenoa repens* extract, obtained with any suitable extraction process, as above disclosed, is suitable to be used according to the present invention. Said lipidosterolic extract typically comprises about 80-95 wt% of total fatty acids, about 0.01-1 wt% of fatty alcohols and about 0.1-1 wt% phytosterols, as main components. The fatty acids can be either as free fatty acids (typically, from about 60 wt% to about 95 wt% of total fatty acids) or as various esters, typically, methyl and ethyl esters and glycerides. The main fatty acids present in the extract include caproic, caprylic, capric, lauric, myristic, palmitic, palmitoleic, stearic, oleic, linoleic and linolenic acid. Lauric acid and oleic acid are typically the major fatty acids, amounting to about 55-80% of total fatty acids; other major fatty acids are myristic, palmitic and linoleic. Sterols include beta-sitosterol, campesterol, and stigmasterol. Major sterol is typically beta-sitosterol. Fatty alcohols generally include octanosanol, hexacosanol, triacontanol, and tetracosanol.

The *Serenoa repens* solid extract is the starting material used for preparing the *Serenoa repens* granules according to the present invention, by roller compaction process. This starting material is also referred to as "regular", or "commercial", or "standard", or "conventional" or "uncompacted" *Serenoa repens* solid extract. On the other hand, the final product, i.e., the *Serenoa repens* extract granules is also designated granular *Serenoa repens* extract.

The *Serenoa repens* solid extract, as meant in the present invention, consists of an oily *Serenoa repens* extract (*Serenoa repens* lipidosterolic extract) and a pharmaceutically acceptable solid carrier. The pharmaceutically acceptable solid carrier is, for example, a carbohydrate, typically selected from maltodextrin, starch, modified starch or alginate. Other suitable pharmaceutically acceptable solid carrier is silica, particularly, colloidal anhydrous silica; or silicates, for example, calcium silicate.

A modified starch, as is well known in the art, is a starch modified by chemical means, for example, acid-modified, bleached, oxidized, esterified, etherified, or treated enzymatically. Suitable modified starches include, among others, pregelatinized starch, hydroxypropyl starch, hydroxypropyl distarch phosphate, monostarch phosphate, distarch phosphate, hydroxyethyl starch, starch acetate, acetylated distarch adipate, starch sodium octenyl succinate, starch aluminium octenyl succinate or carboxymethylated starch.

In an embodiment, the pharmaceutically acceptable solid carrier is selected from maltodextrin, starch, modified starch, alginate, colloidal anhydrous silica and calcium silicate, preferably is selected from maltodextrin, starch, alginate, pregelatinized starch, hydroxypropyl starch, hydroxyethyl starch, starch acetate, starch sodium octenyl succinate, starch aluminium octenyl succinate, carboxymethylated starch, colloidal anhydrous silica and calcium silicate.

In an embodiment, the pharmaceutically acceptable solid carrier is a modified starch, preferably selected from pregelatinized starch, hydroxypropyl starch, hydroxyethyl starch, starch acetate, starch sodium octenyl succinate, starch aluminium octenyl succinate, and carboxymethylated starch.

In a preferred embodiment, the pharmaceutically acceptable solid carrier is starch sodium octenyl succinate (CAS 66829-29-6).

Starch may be of any common origin, namely, maize starch, potato starch, pea starch, rice starch, wheat starch or tapioca starch.

The *Serenoa repens* solid extract typically comprises from about 30 wt% to about 60 wt% of the pharmaceutically acceptable solid carrier, and the remainder up to 100 wt% of oily *Serenoa repens* extract, namely, from about 70 wt% to about 40 wt%. Preferably, the *Serenoa repens* solid extract comprises from about 40 wt% to about 60 wt% of the pharmaceutically acceptable solid carrier and from about 60 wt% to about 40 wt% of oily *Serenoa repens* extract, more preferably comprises from about 45 wt% to about 55 wt% of the pharmaceutically acceptable solid carrier and from about 55 wt% to about 45 wt% of oily *Serenoa repens* extract, and still more preferably comprises about 50 wt% of pharmaceutically acceptable solid carrier and about 50 wt% of *Serenoa repens* oily extract.

The *Serenoa repens* solid extract, comprising the lipidosterolic oily extract and the solid carrier, can be prepared by conventional processes. For example, the oil can be directly poured and adsorbed onto the carrier, or alternatively can be previously dissolved in a suitable solvent, sprayed onto the carrier and dried; or alternatively a slurry or an emulsion can be prepared with the oily extract, the carrier and a suitable solvent and spray dried, or freeze dried.

Within the context of the present invention, therefore, it is understood that the *Serenoa repens* solid extract, consisting of the *Serenoa repens* lipidosterolic extract and the pharmaceutically acceptable solid carrier, may contain minor, residual amounts of some solvent used in its preparation, depending on the preparation method used.

Furthermore, *Serenoa repens* solid extracts as above described, namely, consisting of a *Serenoa repens* lipidosterolic extract adsorbed on or somehow mixed with a solid carrier, are widely commercially available, for example, from the companies Fontana, Solchem Nature, Xi'an Green Spring Technology, or Xi'an Herbsine Bioengineering, among others.

*Serenoa repens* solid extracts, as disclosed above, are generally in the form of hygroscopic and poorly flowable powders. This may be due to the fact that the carrier particles used for their preparation need to be fine, of large surface area, to be able to absorb high load of the *Serenoa repens* oily substance. However, this has the drawback that the obtained solid extracts are not easy to handle and also are not optimal to be processed into pharmaceutical dosage forms. Said solid extracts are particularly unsuitable for the preparation of tablets by direct compression.

As is well-known in the art, the bulk density, also called apparent density, can be defined as the ratio of the mass to the volume of an untapped powder sample, as "poured". The bulk density is given in grams per millilitre (g/ml) and is typically calculated by "pouring" a known mass of powder into a graduated cylinder.

On the other hand, tapped density, also called compacted density, of a powder is the ratio of the mass of the powder to the volume occupied by the powder after it has been tapped for a defined period of time. Tapped density is measured by first gently introducing a known sample mass into a graduated cylinder and carefully levelling the powder without compacting it. The cylinder is then mechanically tapped by raising the cylinder and allowing it to drop under its own weight using a suitable mechanical tapped density tester that provides a suitable fixed drop distance and nominal drop rate.

The bulk (or apparent) density of the uncompacted *Serenoa repens* solid extract, i.e., before being subjected to the process according to the present invention, generally ranges from about 0.20 g/ml to about 0.60 g/ml, preferably from about 0.25 g/ml to 0.55 g/ml, more preferably from about 0.25 g/ml to about 0.50 g/ml, and still more preferably ranges from about 0.30 g/ml to about 0.40 g/ml.

The tapped (or compacted) density of the *Serenoa repens* solid extract typically ranges from about 0.30 g/ml to about 0.65 g/ml, preferably from about 0.40 g/ml to about 0.60 g/ml and more preferably ranges from about 0.45 g/ml to 0.55 g/ml.

*Serenoa repens* solid extract of whatever bulk or tapped density can be used as starting material to prepare the improved *Serenoa repens* granules according to the present invention.

Bulk density and tapped density can be measured according to the method disclosed in the European Pharmacopoeia section 2.9.34 (*Bulk density and tapped density of powders),* for example, the bulk density may be measured using the PT-SV100 Scott Volumeter and the tapped density may be measured using the PT-TD200 Tap Density Tester (Pharma Test Apparatebau AG, Germany).

As is well-known in the art, there are several parameters which can be used to assess the flowability of a powder or granulate, namely, the Carr index, Hausner ratio, flow rate and the angle of repose. Some of these parameters are related to the density of the substance, namely, to its bulk (apparent) density and its tapped (compacted) density.

The Carr index, also called Carr's index or compressibility index, is calculated according to the following formula:

$$Carr\ index = \frac{Density\ (tapped) - Density\ (bulk)}{Density\ (tapped)} \times 100$$

Free flowing powders have smaller values of the Carr index than poor flowing powders. In general, a Carr index greater than 25 is considered to be an indication of poor flowability, and below 15, of good flowability.

Hausner ratio is also predictive of powder flow, and is calculated according to the following formula:

$$Hausner\ ratio = \frac{Density\ (tapped)}{Density\ (bulk)}$$

In this case, a Hausner ratio greater than 1.34 is generally considered to be an indication of poor flowability.

The relationship between Carr index and/or Hausner ratio with powder flowability is well recognized in the art, for example, as disclosed by M. E. Aulton, in "Powder flow", chapter 12 of the well-recognized reference book in pharmaceutical technology "Aulton's Pharmaceutics. The design and manufacture of medicines", Fifth Edition 2018, M.E. Aulton and K.M.G. Taylor, Editors, Elsevier Ltd, as summarized in the following table:

| Type of flow | Carr index | Hausner ratio |
|---|---|---|
| Excellent | 1-10 | 1.00-1.11 |
| Good | 11-15 | 1.12-1.18 |
| Fair | 16-20 | 1.19-1.25 |
| Passable | 21-25 | 1.26-1.34 |
| Poor | 26-31 | 1.35-1.45 |
| Very poor | 32-37 | 1.46-1.59 |
| Very, very poor | >37 | >1.59 |

The "angle of repose" is another parameter for indirectly quantifying powder flowability. It is the angle at which a material will rest on a stationary heap, specifically, is the angle formed by the heap slope and the horizontal plane. It may be calculated by different methods, commonly, just pouring the material on a flat surface. It also can be calculated as "drained angle of repose", as the angle measured on the internal conical face of a material which has been formed when drained from an orifice of a flat-bottomed container to the horizontal. A dynamic angle of repose can also be used, which is the angle to the horizontal of the free surface formed in a relatively slowly rotating drum (Aulton, *op. cit.,* pg. 197).

In this case, the lower the value of the angle of repose, the higher the flowability of the material. It is generally considered that an angle of repose higher than 45 ° correlates with poor flowability, whereas an angle of repose under 30 ° correlates with excellent flowability.

A conventional *Serenoa repens* solid extract, prepared with conventional carrier particles, as discussed above, and as is also typically available commercially, is generally a hygroscopic and lumpy powder, poorly flowable, having agglomerated appearance (see Figure 1) and practically insoluble in water.

For example, the *Serenoa repens* solid extract starting material used in Example 1 has 0.35 g/ml apparent density, 0.48 g/ml tapped density and 5.0% humidity. The compressibility (Carr Index) is 27.08%, while its Hausner ratio value is 1.37.

Both values indicate that *Serenoa repens* extract has poor flowability and, therefore, it is inappropriate for preparing pharmaceutical dosage forms containing it. Its angle of repose is > 45 °, indicating also poor flowability. Accordingly, its handling requires the use of shaking or vibration. Moreover, the preparation of tablets therewith by direct compression is difficult and tablets of poor quality are generally obtained therewith, as disclosed in Tomar *et al., op.cit.*

Uncompacted *Serenoa repens* solid extract used for the preparation of the granular *Serenoa repens* according to the present invention, is typically a powder of fine particle size, with mean particle size generally of less than 800 microns, or less than 700 microns, or less than 600 microns, or less than 500 microns, or less 400 microns, typically the mean particle size is in the range 100-300 microns.

The composition of the conventional *Serenoa repens* solid extract used as starting material is as discussed above, i.e., it consists of the *Serenoa repens* oil extract adsorbed or otherwise mixed with a suitable pharmaceutically active carrier and is not further mixed or combined with other substances, neither with any excipient or vehicle nor with any additional active ingredient before being subjected to the process according to the invention.

Roller compaction

A *Serenoa repens* solid extract, as defined above, is used as starting material to prepare the granular *Serenoa repens* extract according to the present invention.

The process according to the present invention comprises roller compaction of said conventional *Serenoa repens* solid extract.

Roller compaction technology is well-known in the pharmaceutical field and is widely described in different reference manuals in the art, for example, in R.W. Miller, Roller Compaction Technology, in: Handbook of Pharmaceutical Granulation Technology, Editor D.M. Parikh, Third Edition, Informa Healthcare USA, 2010, Chapter 8, 163-182.

Briefly, in the roller compaction process, the powder being compacted is squeezed between two counter-rotating rolls to form a compressed sheet, which is subsequently milled into granules. Therefore, the roller compaction machines, or roller compactors, typically comprise a main roller unit connected with a feeding system, for feeding the starting material to be compressed into the roller unit, and are connected to a granulating/milling system, for milling the compacted sheet exiting from the rollers.

Roller compaction machines are well known in the art and are commercially available through many different companies, for example, Eurotab Bonals, Gerteis Maschinen + Processengineering, Freund Corporation, Alexander-Werk, Powtec, Hosokawa Alpine or L.B. Bohle Maschinen und Verfahrenmany, among many others.

The roller compactors available in the market may be characterized according to several parameters, for example, the type of feeding system, the range of compacting forces, the roll speed range, the diameter, and the width (also referred to as the length) of the rolls, among others.

Some of these parameters can be adjusted during the compaction process in order to optimize the results, for example, the feed speed, roll speed, roll pressure, roll gap and mill speed, as discussed below.

The roller compaction process, therefore, comprises two main steps:

a) Compression step
b) Milling step

*Compression step*

The compression step is performed by passing the substance to be compressed, i.e., the *Serenoa repens* powder extract, through the two counter rotating rollers, to form a compressed sheet.

The compression is performed in the roller unit, which consists of two equal diameter counter rotating rolls through which the powder is passed to get compacted. The two rolls can be mounted in horizontal, vertical or inclined position.

Typically, the roll diameter may range from about 100 mm to about 450 mm, depending on the roller compactor used. The roll diameter of the roller compactor to be used is not crucial, and all are suitable for performing the process of the current invention. For example, roll diameter of 150-250 mm is particularly suitable.

Typically, the roll width (also referred to as roll length) may range from about 20 mm to about 120 mm, and any width may be suitable for performing the method of the present invention. The higher the width of the rollers, the higher the amount of *Serenoa repens* extract that can be processed per unit of time. For example, widths comprised between 50 mm and 100 mm are suitable.

The roll speed can be adjusted and typically may range from about 5 r.p.m to about 35 r.p.m. In one embodiment of the invention the roll speed is comprised between 5 r.p.m. and 20 r.p.m., more preferably comprised between 5 r.p.m. and 15 r.p.m., and still more preferably between 7 r.p.m. and 10 r.p.m., for example, is about 7 r.p.m. or is about 8 r.p.m., or is about 9 r.p.m. or is about 10 r.p.m.

Another adjustable parameter is the roll gap, i.e., the distance between the two rolls. It typically ranges from about 0.2 mm to about 2 mm, preferably from about 0.2 mm to about 1.5 mm, more preferably from about 0.3 mm to about 1.0 mm, and still more preferably from about 0.4 mm to about 0.8 mm. In particular embodiments of the invention, the roll gap is selected from about 0.4 mm, about 0.5 mm, about 0.6 mm, about 0.7 mm and about 0.8 mm. In one embodiment, the roll gap is about 0.4 mm.

Generally, one roll is fixed, while the other is movable and exerts pressure on the fixed roll by means of a hydraulic pressure control system. The horizontal pressure/force between rolls can be also adjusted to optimize the properties of the end-product.

The roller compactors available in the market may provide a wide range of compacting forces, generally ranging from about 10 kN to about 250 kN or higher. Typically, the compacting force is comprised between 50 kN and 200 kN, preferably between 60 kN and 175 kN, more preferably between 100 kN and 160 kN and still more preferably comprised between 135 kN and 150 kN.

The roll force applied during the compression may be suitably expressed, for example, as force per unit of roller length. Typically, the force per unit of roller length in the method of the invention may range from about 2 kN/cm to about 35 kN/cm, preferably from about 6 kN/cm to about 30 kN/cm, more preferably from about 8 kN/cm to about 25 kN/cm, still more preferably from about 14 kN/cm to about 22 kN/cm, and still more preferably from about 18 kN/cm to about 20 kN/cm.

The roll surface may be either smooth or non-smooth, i.e., may have rough pattern surface, for example, fluted, grooved or knurled surface. The roll surface of the two rolls may be identical or different.

In one embodiment of the invention, both rolls have smooth surface. In another embodiment of the invention, one roll has a smooth surface and the other has a non-smooth surface, for example, fluted, grooved, or knurled surface.

The main roller unit is generally connected with a feeding system, whose purpose is to feed the powder to be compacted to the rolls and ensure a uniform flow of material in order to continuously fill the nip between the rolls as they rotate. It can be a gravity feeder, wherein the powder is fed vertically by gravity, or a force feeder, wherein the powder is pushed towards the rolls by one or several screws. Preferably, screw feeding is used in the present invention, and the feeder can be vertical, horizontal, or inclined. In one embodiment, the screw feeder is vertical and is situated in the upper part of the equipment, over the rolls. The screw feeder may be a hopper or analogous container containing an endless screw.

The feed speed can be adjusted, preferably to a constant feed speed, for example, by adjusting the rotation speed of the endless screw. Typically, the screw rotation speed is comprised between about 10 r.p.m. and about 80 r.p.m. In one embodiment of the invention, the screw rotation speed is comprised between 30 r.p.m. and 80 r.p.m., preferably is comprised between 45 r.p.m. and 70 r.p.m., and more preferably comprised between 55 r.p.m. and 65 r.p.m. In one embodiment, the feed speed is about 60 r.p.m.

Typically, *Serenoa repens* solid extract raw material is sent for compaction into the screw feeder from another hopper or reservoir containing the product, for example, using vacuum for sucking the powder and sending it to the screw feeder in a controlled way. The sucking of the raw material and its delivery into the screw feeder can be controlled in an automatic way, for example, by placing a probe inside the feeder that activates the feeding of the raw material when necessary.

*Milling step*

Once the *Serenoa repens* solid extract has been compacted, after passing through the two counter rotating rollers, it is transformed in a compressed sheet or ribbon, which is subsequently sent to the granulation/milling module, which is typically placed under the compacting rollers. Such granulation/milling module comprises one or more mills which break the compacted ribbon into compacted granules.

In this step of the roller compaction process, the speed of the granulation mill can also be adjusted. This speed is not critical, and is typically comprised in the range of from about 5 r.p.m. to about 500 r.p.m, preferably from about 5 r.p.m. to about 200 r.p.m, more preferably from about 10 r.p.m. to about 100 r.p.m, still more preferably from about 10 r.p.m. to about 30 r.p.m, for example is selected from about 10 r.p.m. about 15 r.p.m., about 20 r.p.m., about 25 r.p.m., and about 30 r.p.m., more preferably is about 15 r.p.m.

Typically, after milling, the granules obtained are sieved through the suitable mesh size to obtain granules of the desired particle size.

It is understood that the roller compaction process according to the present invention involves compacting a standard *Serenoa repens* powder extract alone, i.e., without any additional excipient or vehicle.

In one embodiment of the invention, the *Serenoa repens* solid extract is subjected to roller compaction using the following parameters:

- Roll speed comprised between 5 r.p.m. and 35 r.p.m., preferably between 5 r.p.m. and 20 r.p.m., more preferably between 5 r.p.m. and 15 r.p.m. and still more preferably between 7 r.p.m. and 10 r.p.m., for example selected from about 7 r.p.m., about 8 r.p.m., about 9 r.p.m., and about 10 r.p.m.
- Roll gap comprised between 0.2 mm and 2 mm, preferably between 0.2 mm and 1.5 mm, more preferably between 0.3 mm and 1.0 mm, and still more preferably between 0.4 mm and 0.8 mm, for example selected from about 0.4 mm, about 0.5 mm, about 0.6 mm, about 0.7 mm, and about 0.8 mm.
- Roll force between the rolls comprised between 10 kN and 250 kN, preferably between 50 kN and 200 kN, more preferably between 60 kN and 175 kN, still more preferably between 100 kN and 160 kN, and still more preferably comprised between 135 kN and 150 kN; and/or roll force per unit of roller length comprised between 2 kN/cm and 35 kN/cm, preferably between 6 kN/cm and 30 kN/cm, more preferably between 8 kN/cm and 25 kN/cm, still more preferably between 14 kN/cm and 22 kN/cm, and still more preferably comprised between 18 kN/cm and 20 kN/cm;

wherein preferably:

- the screw rotating speed of the feeding system is comprised between 10 r.p.m. and 80 r.p.m., more preferably between 30 r.p.m. and 80 r.p.m., still more preferably between 45 r.p.m. and 70 r.p.m, still more preferably between 55 r.p.m. and 65 r.p.m., and still more preferably is about 60 r.p.m; and/or
- the speed of the granulation mill is comprised between 5 r.p.m. and 500 r.p.m., preferably between 5 r.p.m. and 200 r.p.m., more preferably between 10 r.p.m. and 100 r.p.m., and still more preferably between 10 r.p.m. and 30 r.p.m., for example, is selected from about 10 r.p.m., about 15 r.p.m., about 20 r.p.m., about 25 r.p.m., and about 30 r.p.m., more preferably is about 15 r.p.m.

Granular *Serenoa repens* extract

The granular *Serenoa repens* extract obtainable with the process according to the present invention shows outstandingly improved flowability and tabletting performance compared to the standard *Serenoa repens* powder extract.

As discussed above, the main parameters used to assess the flowability of a powder or granulate are Carr index, Hausner ratio, flow rate and the angle of repose.

As disclosed in Example 2, the granular *Serenoa repens* extract obtained according to the process of the present invention has excellent flowability and optimal properties for direct compression, as shown by the values of the Carr index, Hausner ratio and the angle of repose.

In particular, the method of the invention allows obtaining granular *Serenoa repens* extract having a Carr index generally lower than 12, preferably lower than 10, more preferably lower than 8, still more preferably lower than 7, and still more preferably lower than 6.5, for example comprised between 4 and 6, preferably comprised between 4 and 5.

Analogously, the method of the invention allows obtaining granular *Serenoa repens* extract having a Hausner ratio lower than 1.25, preferably lower than 1.20, more preferably lower than 1.18, still more preferably lower than 1.14, still more preferably lower than 1.12, still more preferably equal to or lower than 1.11, namely, comprised between 1.00 and 1.11.

As shown in Example 2, using the method of the present invention, it is possible to obtain a granular *Serenoa repens* extract having the same chemical composition than a standard *Serenoa repens* solid extract, but where the flowability parameters are outstandingly improved, in particular, the compressibility index is lowered from 27.08% in the standard *Serenoa repens* powder extract to 4.22% in the granular *Serenoa repens;* analogously, the Hausner ratio is lowered from 1.37 in the starting material to 1.04 in the granular end product.

Furthermore, regular *Serenoa repens* solid extract, when subjected to the process according to the present invention, undergoes a significant increase of its density. As shown in Example 2, for example, the bulk density increases in about 94 %, from a bulk density of about 0.35 g/ml of the starting powder extract to a bulk density of about 0.66 g/ml of the granular *Serenoa repens* extract.

The method of the invention, therefore, allows obtaining granular *Serenoa repens* extract having bulk (or apparent) density ranging from about 0.55 g/ml to about 0.80 g/ml, preferably from about 0.60 g/ml to 0.85 g/ml, more preferably from about 0.65 g/ml to about 0.80 g/ml, and still more preferably ranges from about 0.65 g/ml to about 0.75 g/ml.

The tapped (or compacted) density of the granular *Serenoa repens* extract of the present invention typically ranges from about 0.60 g/ml to about 0.90 g/ml, preferably from about 0.65 g/ml to about 0.80 g/ml and more preferably ranges from about 0.70 g/ml to 0.80 g/ml.

Therefore, the granular *Serenoa repens* extract obtained with the process of the present invention is advantageous for manufacturing pharmaceutical compositions due to its improved rheological properties, so it flows easily and does not stick to the walls of the containers during its handling and during the manufacturing process of said compositions, avoiding the need, for example, of using shaking or vibration. Furthermore, as shown in Example 3, using the granular extract obtained according to the method of the present invention, it is possible to prepare *Serenoa repens* tablets by simple direct compression, using conventional excipients and having high load of active ingredient.

Additionally, an improvement in the solubility of the extract was also achieved with the process of the invention. Indeed, it was surprisingly found that the granular *Serenoa repens* extract according to the present invention is moderately soluble in water, whereas the original powder extract is practically insoluble.

Therefore, another aspect of the invention is the granular *Serenoa repens* extract obtainable with the method of the present invention.

As discussed above, the size of the granular *Serenoa repens* extract is generally adjusted after the milling step in the roller compaction process, typically, by sieving the granules obtained after milling the compressed sheet or ribbon. The granules are sieved, using a sieve of suitable mesh size to obtain granules of the desired size, namely, under a specific size determined by the sieve opening.

Typically, the mesh size of the sieve is adjusted to obtain particles of less than 2000 microns, preferably less than 1800 microns, preferably less than 1600 microns, more preferably less than 1400 microns, still more preferably less than 1200 microns, still more preferably less than 1000 microns, still more preferably less than 900 microns, and still more preferably less than 800 microns. The particle size, for example, is typically comprised between 400 and 2000 microns, preferably comprised between 400 and 1000 microns, and more preferably comprised between 500 and 800 microns.

Pharmaceutical compositions

As discussed above, the granular *Serenoa repens* extract obtained with the process of the present invention, has improved flowability and compressibility, and can be advantageously used for the preparation of pharmaceutical compositions.

Hence, the improved flowability is an advantage for the manufacturing of pharmaceutical compositions, as the product flows more easily, does not stick to the walls of vessels or blenders, or to the punches and dies of tableting machines, for example, therefore avoiding product loses and improving the equipment maintenance. Furthermore, the improved flowability of granular *Serenoa repens* extract is also an advantage for preparing powder pre-mixtures with excipients, for example, for manufacturing tablets or capsules, avoiding segregation of the components and providing better homogeneity of the blend.

Additionally, the improved compressibility (related to its low Carr index) of granular *Serenoa repens* extract enables the preparation of tablets of optimal properties (such as hardness, friability, weight uniformity and disintegration time) by direct compression.

Therefore, another aspect of the invention is the use of the granular *Serenoa repens* extract of the present invention for the preparation of a pharmaceutical composition, preferably a solid pharmaceutical composition.

Another aspect of the invention is a pharmaceutical composition, preferably a solid pharmaceutical composition, comprising the granular *Serenoa repens* extract of the present invention and at least one pharmaceutically acceptable excipient and/or vehicle.

Another aspect of the invention is a process for preparing a pharmaceutical composition, preferably a solid pharmaceutical composition, comprising *Serenoa repens* as active ingredient, comprising mixing granular *Serenoa repens* according to the present invention and at least one pharmaceutically acceptable excipient and/or vehicle.

Optionally, the pharmaceutical composition comprising the granular *Serenoa repens* extract can contain one or more additional active ingredients, for example, other herbal extracts, to reinforce the desired activity of the composition.

Typically, the pharmaceutical composition comprising granular *Serenoa repens* extract as active ingredient, according to the present invention can be any conventional formulation which can be prepared using methods that are well known to the person skilled in pharmaceutical formulation, as those disclosed in handbooks of pharmaceutical technology, for example, in the book J.P. Remington and A. R. Genaro, Remington The Science and Practice of Pharmacy, 20th edition, Lippincott, Williams & Wilkins, Philadelphia, 2000 [ISBN: 0-683-306472] or in the book M.E. Aulton and K.M.G. Taylor, Aulton's Pharmaceutics, the design and manufacture of medicines, 5th edition, Elsevier Ltd, 2018 [ISBN: 978-0-7020-7005-1], or in the book A.K. Dash, S. Singh and J. Tolman, Pharmaceutics. Basic principles and application to pharmacy practice, Academic Press, Elsevier, 2014 [ISBN: 978-0-12-386890-9].

The excipients suitable to be used in the pharmaceutical compositions of the present invention are also well known to those skilled in pharmaceutical technology and are described, for example, in the book R.C. Rowe, P.J. Sheskey and P.J. Weller, Handbook of Pharmaceutical Excipients, Sixth Edition, Pharmaceutical Press, 2009.

For the purpose of the present description, all compositions of *Serenoa repens* extract disclosed herein are referred to, in general, as "pharmaceutical compositions", even if they, for regulatory or other reasons, might be considered a dietary supplement, rather than a medication. It is noted that, in general, the components and manufacturing processes of the formulations are substantially the same, irrespective that the final product can be classified either as a dietary supplement or as a medication. Therefore, along the present description, as well as in the claims, the term "pharmaceutical composition" may be replaced with the term "dietary composition", when the composition may be classified as dietary supplement.

The pharmaceutical compositions comprising the granular *Serenoa repens* extract are typically solid pharmaceutical compositions for oral administration, preferably, selected from tablets and capsules.

In an embodiment, the pharmaceutical composition is a tablet.

Tablets comprising the granular *Serenoa repens* extract of the present invention can be formulated using standard procedures, well known in the art. Preferably, tablets are prepared by direct compression taking advantage of the good compressibility of granular *Serenoa repens* extract, i.e., the granular extract is mixed with suitable excipients and compressed without any previous granulation process.

A particular aspect of the present invention is a process for preparing a tablet comprising *Serenoa repens* as active ingredient, comprising mixing the granular *Serenoa repens* according to the present invention and at least one pharmaceutically acceptable excipient and/or vehicle and preparing the tablet by direct compression.

Excipients typically used in the formulation of tablets are diluents, binding agents, glidants, lubricants, disintegrating agents, and mixtures thereof. Other additional excipients include sweetening agents, flavouring agents and colouring agents, for example.

Suitable diluents include calcium carbonate, calcium phosphate, calcium sulfate, cellulose acetate, dextrates, dextrins, dextrose, ethylcellulose, fructose, glyceryl palmitostearate, isomalt, kaolin, lactitol, lactose, magnesium carbonate, magnesium oxide, maltodextrins, maltose, mannitol, microcrystalline or powdered cellulose, pregelatinized starch, starch, sodium carbonate, sodium chloride, sorbitol and sucrose, among others, and mixtures thereof.

Suitable binding agents include acacia, agar, cellulose acetate phthalate, copovidone, dextrates, dextrin, dextrose, ethylcellulose, gelatin, glyceryl behenate, guar gum, hydroxyethyl cellulose, hydroxyethylmethyl cellulose, hydroxypropyl cellulose, hydroxypropylmethyl cellulose, methylcellulose, maltodextrin, microcrystalline cellulose, povidone, pregelatinized starch, sodium carboxymethylcellulose, starch, stearic acid and sucrose, among others, and mixtures thereof.

Suitable glidants include powdered cellulose, colloidal silicon dioxide, magnesium oxide, magnesium silicate, magnesium trisilicate, silicon dioxide, and talc, among others, and mixtures thereof.

Suitable lubricants include calcium stearate, glyceryl behenate, glyceryl palmitostearate, hydrogenated castor oil, magnesium stearate, myristic acid, palmitic acid, polyethylene glycol, sodium benzoate, sodium lauryl sulfate, sodium stearyl fumarate, stearic acid, and talc, among others, and mixtures thereof.

Suitable disintegrants include alginic acid, crospovidone, sodium croscarmellose, sodium starch glycolate, starch, and low-substituted hydroxypropyl cellulose, among others, and mixtures thereof.

Furthermore, tablets may be formulated either as conventional compressed tablets, or in other forms, for example, buccal, sublingual, chewable, sustained-release or orally-disintegrating tablets, by selecting suitable excipients and manufacturing processes, as are well known in the art.

In another embodiment, the pharmaceutical composition is a capsule, preferably a hard capsule.

Capsules, as is well known in the pharmaceutical field, are solid dosage forms in which the active substance is enclosed in either a hard or soft, soluble container or shell. The raw materials used in the manufacture of the shell are similar for both hard and soft gelatin capsules, while the proportions vary. The major component of a capsule shell is typically gelatin; other components include water, plasticizers, such as glycerol, sorbitol or propylene glycol, colorants, opacifiers, such as titanium dioxide, and preservatives. Hypromellose can alternatively be used as capsule shell material for hard capsules.

Hard capsules, in particular, consist of two sections, one slipping over the other and thus enclosing the drug formulation, which is generally in solid form, as powder or granulate.

The powders or granules included within hard capsules comprise the *Serenoa repens* active ingredient and typically at least one pharmaceutically acceptable excipient, generally selected from diluents, lubricants and glidants, and mixtures thereof, which are well-known in the art, for example, those disclosed above for the formulation of tablets.

In oral dosage forms, namely in capsules or tablets, each unit dose typically comprises from about 200 to about 1000 mg, preferably from about 300 to about 800 mg of granular *Serenoa repens*.

Use

Another aspect of the present invention relates to a pharmaceutical composition comprising the granular *Serenoa repens* extract of the present invention for use in therapy.

Another aspect of the invention is a method of treating a disease or condition susceptible to treatment with *Serenoa repens* extract in a subject, the method comprising administering a pharmaceutical composition comprising the granular *Serenoa repens* extract of the present invention to the subject.

The terms "treatment", "treating" or "therapy" as used herein have their conventional meaning and typically refer to the administration of the pharmaceutical composition with the purpose of attenuating, alleviating, minimising, inhibiting the progression, or suppressing the symptoms of the treated disease or condition, or for stimulating the recovery after said disease or condition. Furthermore, the treatment is also meant to include the preventive aspect, i.e., the administration of the granular *Serenoa repens* containing pharmaceutical composition to a subject suspected or prone to suffer from a specific disease or condition in order to prevent the onset of such disease or condition. The pharmaceutical composition comprising granular *Serenoa repens* for use in therapy, according to this aspect of the invention, is meant therefore to include the treatment and/or prevention of any disease or condition susceptible to be treated or prevented with *Serenoa repens* extract.

Main therapeutic indication of *Serenoa repens* extract is the treatment of symptoms of benign prostatic hyperplasia (BPH), in particular, for alleviation of micturition disorders, such as dysuria, pollakiuria, nocturia and urine retention.

The therapeutically effective dose of *Serenoa repens* extract is generally comprised between 100 and 500 mg daily of *Serenoa repens* lipidosterolic extract, more preferably comprised between 200 mg and 400 mg, and still more preferably is about 320 mg daily of *Serenoa repens* lipidosterolic extract.

The present invention involves the following embodiments:

1.- A process for the preparation of *Serenoa repens* extract granules comprising roller compaction of a *Serenoa repens* solid extract.

2.- Process according to embodiment 1, characterized in that the *Serenoa repens* solid extract consists of an oily *Serenoa repens* lipidosterolic extract and a pharmaceutically acceptable solid carrier.

3.- Process according to embodiment 2, characterized in that the pharmaceutically acceptable solid carrier is selected from maltodextrin, starch, modified starch, alginate, colloidal anhydrous silica and calcium silicate.

4.- Process according to embodiment 3, characterized in that the pharmaceutically acceptable solid carrier is a modified starch, preferably selected from pregelatinized starch, hydroxypropyl starch, hydroxyethyl starch, starch acetate, starch sodium octenyl succinate, starch aluminium octenyl succinate, and carboxymethylated starch; and more preferably is starch sodium octenyl succinate.

5.- The process according to any one of embodiments 2 to 4, characterized in that the oily *Serenoa repens* lipidosterolic extract is obtained by extraction with n-hexane, ethanol or supercritical $CO_2$, preferably by extraction with n-hexane or supercritical $CO_2$, and more preferably by extraction with supercritical $CO_2$.

6.- The process according to any one of embodiments 2 to 5, characterized in that the *Serenoa repens* solid extract comprises from 30 wt% to 60 wt% of the pharmaceutically acceptable solid carrier and from 70 wt% to 40 wt% of the *Serenoa repens* lipidosterolic extract, preferably comprises from 40 wt% to 60 wt% of the pharmaceutically acceptable solid carrier and from 60 wt% to 40 wt% of the *Serenoa repens* lipidosterolic extract, more preferably comprises from 45 wt% to 55 wt% of the pharmaceutically acceptable solid carrier and from 55 wt% to 45 wt% of the *Serenoa repens* lipidosterolic extract, and still more preferably comprises about 50 wt% of the pharmaceutically acceptable solid carrier and about 50 wt% of *Serenoa repens* lipidosterolic extract.

7.- The process according to any one of embodiments 1 to 6, characterized in that the *Serenoa repens* solid extract starting material has a bulk density comprised between 0.20 g/ml and 0.60 g/ml, preferably comprised between 0.25 g/ml and 0.55 g/ml, more preferably comprised between 0.25 g/ml and 0.50 g/ml, and still more preferably comprised between 0.30 g/ml and 0.40 g/ml.

8.- The process according to any one of embodiments 1 to 7, characterized in that the *Serenoa repens* solid extract starting material has a tapped density comprised between 0.30 g/ml and 0.65 g/ml, preferably comprised between 0.40 g/ml and 0.60 g/ml and more preferably comprised between 0.45 g/ml and 0.55 g/ml.

9.- The process according to any one of embodiments 1 to 8, characterized in that the *Serenoa repens* solid extract has a mean particle size of less than 800 microns, preferably less than 700 microns, more preferably less than 600 microns, still more preferably less than 500 microns, still more preferably less 400 microns, and still more preferably comprised between 100 microns and 300 microns.

10.- The process according to any one of embodiments 1 to 9, characterized in that the roll speed is adjusted to a value comprised between 5 r.p.m. and 20 r.p.m., preferably comprised between 5 r.p.m. and 15 r.p.m., and more preferably comprised between 7 r.p.m. and 10 r.p.m.

11.- The process according to any one of embodiments 1 to 10, characterized in that the gap between the rollers is adjusted to a value comprised between 0.2 mm and 2 mm, preferably comprised between 0.2 mm and 1.5 mm, more preferably comprised between 0.3 mm and 1.0 mm, and still more preferably comprised between 0.4 mm and 0.8 mm.

12.- The process according to any one of embodiments 1 to 11, characterized in that the roll force is adjusted to a value comprised between 50 kN and 200 kN, preferably comprised between 60 kN and 175 kN, more preferably comprised between 100 kN and 160 kN, and still more preferably comprised between 135 kN and 150 kN.

13.- The process according to any one of embodiments 1 to 12, characterized in that the roll force per unit of roller length is adjusted to a value comprised between 2 kN/cm and 35 kN/cm, preferably comprised between 6 kN/cm and 30 kN/cm, more preferably comprised between 8 kN/cm and 25 kN/cm, still more preferably comprised between 14 kN/cm and 22 kN/cm, and still more preferably comprised between 18 kN/cm and 20 kN/cm.

14.- The process according to any one of embodiments 1 to 13, characterized in that the speed of the granulation mill is comprised between 5 r.p.m. and 500 r.p.m, preferably comprised between 5 r.p.m. and 200 r.p.m., more preferably comprised between 10 r.p.m. and 100 r.p.m, and still more preferably comprised between 10 r.p.m. and 30 r.p.m.

15.- Granular *Serenoa repens* extract which is obtainable by a process according to any one of embodiments 1 to 14.

16.- Granular *Serenoa repens* extract according to embodiment 15, characterized in that it has a Carr index lower than 12, preferably lower than 10, more preferably lower than 8, still more preferably lower than 7, and still more preferably lower than 6.5, for example comprised between 4 and 6, preferably comprised between 4 and 5.

17.- Granular *Serenoa repens* extract according to embodiments 15 or 16, characterized in that it has Hausner ratio lower than 1.25, preferably lower than 1.20, more preferably lower than 1.18, still more preferably lower than 1.14, still more preferably lower than 1.12, still more preferably equal to or lower than 1.11, for example comprised between 1.00 and 1.11.

18.- Granular *Serenoa repens* extract according to any one of embodiments 15 to 17, characterized in that it has a bulk density comprised between 0.55 g/ml and 0.80 g/ml, preferably comprised between 0.60 g/ml and 0.85 g/ml, more comprised between 0.65 g/ml and 0.80 g/ml, and still more preferably comprised between 0.65 g/ml and 0.75 g/ml.

19.- Granular *Serenoa repens* extract according to any one of embodiments 15 to 18, characterized in that it has a tapped density comprised between 0.60 g/ml and 0.90 g/ml, preferably comprised between 0.65 g/ml and 0.80 g/ml and more preferably comprised between 0.70 g/ml and 0.80 g/ml.

20.- Granular *Serenoa repens* extract according to any one of embodiments 15 to 19, characterized in that it has a particle size comprised between 400 and 2000 microns, preferably comprised between 500 and 800 microns.

21.- Use of the granular *Serenoa repens* extract according to any one of embodiments 15 to 20 for the preparation of a pharmaceutical composition.

22.- Pharmaceutical composition comprising the granular *Serenoa repens* extract according to any one of embodiments 15 to 20 and at least one pharmaceutically acceptable excipient and/or vehicle.

23.- Process for the preparation of a pharmaceutical composition, comprising mixing the granular *Serenoa repens* extract according to any one of embodiments 15 to 20 and at least one pharmaceutically acceptable excipient and/or vehicle.

24.- The use according to embodiment 21 or the pharmaceutical composition according to embodiment 22 or the process according to embodiment 23, characterized in that the pharmaceutical composition is a solid pharmaceutical composition for oral administration, preferably, selected from a tablet and a capsule, more preferably selected from a tablet and a hard capsule.

25.- The use according to embodiment 21 or the pharmaceutical composition according to embodiment 22 or the process according to embodiment 23, characterized in that the pharmaceutical composition is a tablet prepared by direct compression.

26.- Pharmaceutical composition according to any one of embodiments 22 to 25 for use in therapy.

27.- Pharmaceutical composition for use according to embodiment 26, characterized in that it is for use in the treatment of symptoms of benign prostatic hyperplasia (BPH), preferably for the treatment of micturition disorders such, more preferably selected from dysuria, pollakiuria, nocturia and urine retention.

### *Examples*

Example 1.- Preparation of granular *Serenoa repens* extract according to the invention

For the preparation of the granular *Serenoa repens* extract, a commercially available solid extract was used (Xi'an Herbsine Bioengineering Co, China), which was a white fine powder, having particle size defined as 95% pass through 60 mesh (250 micron), comprising 50 wt% of oily extract and 50 wt% of solid carrier. The extraction method was by supercritical $CO_2$ extraction, the solid carrier was a modified starch (sodium starch octenyl succinate, CAS 66829-29-6), and the extract was prepared by mixing the oily extract, the carrier and water, and spray drying. The total fatty acids specification in the product was > 45.0%.

This *Serenoa repens* powder extract had a bulk density of 0.35 g/ml.

For the compaction process, a roller compactor equipment was used (Bonals BC-150/75V), with vacuum system for feeding the raw material into the screw feeding system and with a refrigerating system. The roller compactor had one roll with smooth surface and one roll with knurled surface. The dimensions of both rolls were 10.5 cm diameter and 7.4 cm width.

The roll speed in the roller compactor was set to about 7 r.p.m. Screw feeding speed was 60 r.p.m., roll force was set to 137-147 kN and the roll gap was fixed to 0.4 mm.

The compactor was connected to a rotor granulator for milling the outcoming compacted plaque. The granulation speed was fixed to 15 r.p.m. and the obtained granulate was passed through a sieve of 800 micron opening.

The granular *Serenoa repens* obtained had a bulk density of 0.68 g/ml.

Example 2.- Flowability parameters of the granular *Serenoa repens* prepared in Example 1

The flowability parameters of the original powder extract and the granular *Serenoa repens* obtained with the process of the invention are compared in the following table:

| Property | Powder *Serenoa repens* (starting material) | Granular *Serenoa repens* (Example 1) |
|---|---|---|
| Aspect | White, hygroscopic, and poorly flowable fine powder | Wife granules, highly flowable and with dry appearance |
| Bulk density | 0.35 g/ml | 0.68 g/ml |
| Tapped density | 0.48 g/ml | 0.71 g/ml |
| Compressibility (Carr Index) | 27.08% | 4.22% |
| Hausner ratio | 1.37 | 1.04 |
| Flow rate | Low | Excellent |
| Humidity | 5.0% | 2.5% |
| Angle of repose | >45 ° | <30 ° |

The bulk density was measured using a PT-SV100 Scott Volumeter (Pharma Test) and the tapped density was measured using a PT-TD200 Tap Density Tester (Pharma Test).

The angle of repose was determined by measuring the radius and the height of the cone formed after pouring the powder, using the formula $\tan^{-1}$ (height/radius).

Figure 1 shows a picture of *Serenoa repens* powder extract starting material and Figure 2 shows a picture of *Serenoa repens* granules after the process of the invention. It can be observed that the starting powder extract has damp and agglomerated appearance, whereas the obtained granules have dry, non-agglomerated, flowable appearance.

Furthermore, an increase in the solubility of the *Serenoa repens* extract was also found, as the starting powder extract was practically insoluble, while the obtained granules were moderately soluble.

Example 3.- Preparation of tablets by direct compression with the granulated *Serenoa repens* extract

*Serenoa repens* tablets were prepared using the ingredients listed in the following table:

| Ingredient | Weight % |
|---|---|
| *Serenoa repens* granules (Example 1) | 50 |
| Microcrystalline cellulose | 20 |
| Starch | 14 |
| Hydroxypropyl methyl cellulose | 10 |
| Croscarmellose sodium | 3 |
| Magnesium stearate | 3 |

All ingredients were mixed in Turbula mixer during about 15 minutes and compressed into tablets.

The obtained tablets had the following characteristics:

- Hardness: 50-60 N
- Friability (%): < 0.03
- Weight uniformity: < ± 3%
- Disintegration time: < 1 min

**Claims**

1. A process for the preparation of *Serenoa repens* extract granules comprising roller compaction of a *Serenoa repens* solid extract.

2. Process according to claim 1, **characterized in that** the *Serenoa repens* solid extract consists of an oily *Serenoa repens* lipidosterolic extract and a pharmaceutically acceptable solid carrier.

3. Process according to claim 2, **characterized in that** the pharmaceutically acceptable solid carrier is selected from maltodextrin, starch, modified starch, alginate, colloidal anhydrous silica and calcium silicate.

4. The process according to claims 2 or 3, **characterized in that** the oily *Serenoa repens* lipidosterolic extract is obtained by extraction with n-hexane, ethanol or supercritical $CO_2$, preferably by extraction with n-hexane or supercritical $CO_2$, and more preferably by extraction with supercritical $CO_2$.

5. The process according to any one of claims 2 to 4, **characterized in that** the *Serenoa repens* solid extract comprises from 30 wt% to 60 wt% of the pharmaceutically acceptable solid carrier and from 70 wt% to 40 wt% of the *Serenoa repens* lipidosterolic extract.

6. The process according to any one of claims 1 to 5, **characterized in that** the roll force per unit of roller length is adjusted to a value comprised between 2 kN/cm and 35 kN/cm, preferably comprised between 6 kN/cm and 30 kN/cm, more preferably comprised between 8 kN/cm and 25 kN/cm, still more preferably comprised between 14 kN/cm and 22 kN/cm, and still more preferably comprised between 18 kN/cm and 20 kN/cm.

7. Granular *Serenoa repens* extract which is obtainable by a process according to any one of claims 1 to 6.

8. Granular *Serenoa repens* extract according to claim 7, **characterized in that** it has a Carr index lower than 12, preferably lower than 10, more preferably lower than 8, still more preferably lower than 7, and still more preferably lower than 6.5, for example comprised between 4 and 6, preferably comprised between 4 and 5.

9. Granular *Serenoa repens* extract according to claims 7 or 8, **characterized in that** it has Hausner ratio lower than 1.25, preferably lower than 1.20, more preferably lower than 1.18, still more preferably lower than 1.14, still more preferably lower than 1.12, still more preferably equal to or lower than 1.11, for example comprised between 1.00 and 1.11.

10. Use of the granular *Serenoa repens* extract according to any one of claims 7 to 9 for the preparation of a pharmaceutical composition.

11. Pharmaceutical composition comprising the granular *Serenoa repens* extract according to any one of claims 7 to 9 and at least one pharmaceutically acceptable excipient and/or vehicle.

12. Process for the preparation of a pharmaceutical composition, comprising mixing the granular *Serenoa repens* extract according to any one of claims 7 to 9 and at least one pharmaceutically acceptable excipient and/or vehicle.

13. The use according to claim 10 or the pharmaceutical composition according to claim 11 or the process according to claim 12, **characterized in that** the pharmaceutical composition is a solid pharmaceutical composition for oral administration, preferably, selected from a tablet and a capsule, more preferably selected from a tablet and a hard capsule.

14. The use according to claim 10 or the pharmaceutical composition according to claim 11 or the process according to claim 12, **characterized in that** the pharmaceutical composition is a tablet prepared by direct compression.

15. Pharmaceutical composition according to claims 11, 13 or 14 for use in therapy, preferably in the treatment of symptoms of benign prostatic hyperplasia (BPH), preferably for the treatment of micturition disorders such, more preferably selected from dysuria, pollakiuria, nocturia and urine retention.

FIGURE 1

FIGURE 2

Europäisches
Patentamt

European
Patent Office

Office européen
des brevets

## EUROPEAN SEARCH REPORT

**Application Number**

EP 24 38 2505

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 990 062 A1 (ASAHI BREWERIES LTD [JP]) 12 November 2008 (2008-11-12) * page 4, paragraph 0017 * * page 5, paragraph 0024-0029 * * page 5, paragraph 0031 - page 8, paragraph 0050; examples 1-10 * * claims * | 7,10-15 | INV. A61K9/16 A61K9/20 A61K36/889 |
| X | US 2006/068037 A1 (HARVEY BRYCE M [US] ET AL) 30 March 2006 (2006-03-30) * page 1, paragraph 0003 * * page 2, paragraph 0017-0019 * * page 5, paragraph 0040-0042 * * page 6, paragraph 0052; figure 1 * * example 2 * * claims * | 1-15 | |
| A,D | GB 2 343 452 A (ESSENTIAL NUTRITION LTD [GB]) 10 May 2000 (2000-05-10) * page 3, paragraph 3 * * pages 5-7 * * claims * | 1-15 | |

TECHNICAL FIELDS
SEARCHED (IPC)

A61K

The present search report has been drawn up for all claims

| Place of search | Date of completion of the search | Examiner |
|---|---|---|
| The Hague | 24 October 2024 | van de Wetering, P |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 38 2505

This annex lists the patent family members relating to the patent documents cited in the above-mentioned European search report.
The members are as contained in the European Patent Office EDP file on
The European Patent Office is in no way liable for these particulars which are merely given for the purpose of information.

24-10-2024

| Patent document cited in search report | | Publication date | Patent family member(s) | | Publication date |
|---|---|---|---|---|---|
| EP 1990062 | A1 | 12-11-2008 | CA | 2643054 A1 | 30-08-2007 |
| | | | CN | 101384281 A | 11-03-2009 |
| | | | EP | 1990062 A1 | 12-11-2008 |
| | | | JP | WO2007097333 A1 | 16-07-2009 |
| | | | KR | 20080110993 A | 22-12-2008 |
| | | | US | 2010285157 A1 | 11-11-2010 |
| | | | WO | 2007097333 A1 | 30-08-2007 |
| US 2006068037 | A1 | 30-03-2006 | NONE | | |
| GB 2343452 | A | 10-05-2000 | NONE | | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012017022 A
- EP 250953 A
- GB 2343452 A
- WO 2023023459 A
- FR 2480754 A

### Non-patent literature cited in the description

- Saw Palmetto. Herbal Medicines. Pharmaceutical Press, 2007, 521-529
- **ANNUNZIATA et al.** Saw Palmetto (Serenoa repens. *Nonvitamin and Nonmineral Nutritional Supplements*, 401-402
- **LOPEDOTA et al.** From oil to microparticulate by prilling technique: Production of polynucleate alginate beads loading Serenoa repens oil as intestinal delivery systems. *Int. J. Pharm.*, 2021, vol. 599, 120412
- **TOMAR et al.** Hicel™ silicified microcrystalline cellulose, versatile excipient for nutraceutical herbal tablet formulation. *Int. J. Dev. Res.*, 2019, vol. 9 (6), 28009-104
- **HOOK**. *Eur. Pharm*, 2012
- **W. BARTRAM**. Small, fructus. *Final. European Medicines Agency*, 24 November 2015
- **HÄNSEL et al.** Hagers Handbuch der Pharmazeutischen Praxis: Drogen P-Z. Springer-Verlag, 1994
- **HAM et al.** A study of the physical and chemical properties of saw palmetto berry extract. *Science Direct Working Paper No S1574-0331*, vol. 04, 70214-3
- *CHEMICAL ABSTRACTS*, 66829-29-6
- Powder flow. **M. E. AULTON**. Aulton's Pharmaceutics. The design and manufacture of medicines. Elsevier Ltd, 2018
- Roller Compaction Technology. **R.W. MILLER**. Handbook of Pharmaceutical Granulation Technology. Informa Healthcare, 2010, 163-182
- **J.P. REMINGTON** ; **A. R. GENARO**. Remington The Science and Practice of Pharmacy. Lippincott, Williams & Wilkins, 2000
- **M.E. AULTON** ; **K.M.G. TAYLOR**. Aulton's Pharmaceutics, the design and manufacture of medicines. Elsevier Ltd, 2018
- **A.K. DASH** ; **S. SINGH** ; **J. TOLMAN**. Pharmaceutics. Basic principles and application to pharmacy practice. Academic Press, Elsevier, 2014
- **R.C. ROWE** ; **P.J. SHESKEY** ; **P.J. WELLER**. Handbook of Pharmaceutical Excipients. Pharmaceutical Press, 2009